# EUROPEAN PATENT APPLICATION

(11) **EP 2 500 027 A1**
(43) Date of publication of application: **19.09.2012**
(21) Application number: 10830081.5
(22) Date of filing: 20.04.2010
(51) Int. Cl.: A61K 36/81, A61P 25/34, A61K 36/74, A23L 1/30

(54) **COMPOSITION FOR COUNTERACTING SMOKING TOXICITY**

(30) Priority: 11.11.2009 KR 20090108686
(71) Applicant: Nam, Jong Hyun, Songpa-gu, Seoul 138-110 (KR)
(72) Inventor: Nam, Jong Hyun, Songpa-gu, Seoul 138-110 (KR)
(74) Representative: Isarpatent
(86) International application number: PCT/KR2010/002456
(87) International publication number: WO 2011/059148

(57) **Abstract**

The present invention relates to a composition for counteracting the toxicity of smoking. More particularly, the present invention relates to a composition including an extract from a whole tomato plant *Hedyotis diffusa,* Sorbus commixta, *Lithospermum erythrorhizon* Siebold & Zucc, and Alnus japonica Steud as an active ingredient counteracting the toxicity of smoking.

## Description

### Technical Field

The present invention relates to a composition for counteracting the toxicity of smoking. More particularly, the present invention relates to a composition including an extract from a whole tomato plant and/or other herbs as an active ingredient counteracting the toxicity of smoking.

### Background Art

Smoking is a known risk factor for six of the eight leading causes of death in the world. Unless unchecked, the tobacco epidemic is expected to kill more than 8 million people a year by 2030 (Mpower, 2008, WHO report on the global tobacco epidemic, WHO).

The smoking prevalence among South Korean adults, particularly among adult males, amounts to 52.3% as of 2005 which is high compared to other countries. It ranks 13^{th} out of 132 WHO member states for which data is obtainable from (Mpower, 2008, WHO report on the global tobacco epidemic, WHO) and ranks first among the Organization for Economic Cooperation and Development (OECD) countries (OECD health data, 2006).

Smoking causes a number of serious diseases including lung cancer, chronic obstructive pulmonary disease (COPD), coronary artery disease, apoplexy (cerebrovascular disease), heart failure, aortic aneurysm, cardiovascular disease (peripheral vascular disease, etc.), laryngopharyngeal cancer, oral cavity cancer, and the like (Yoon, Seok Jun et al., Journal of Preventive Medicine and Public Health 34(3), 2001, "Estimation of Attributable Burden due to Premature Death from Smoking in Korea").

As many as 4,000 toxic materials including carcinogens are found in tobacco smoke. Representative among them are tar and nicotine.

Tar in cigarettes contains 2,000 toxic chemical species including 20 carcinogens including dioxin, benzopyrene, dimethylnitrosamine, etc. (Shin D C, Toxic Chemicals in Tobacco Smoke, published by the Korean Association of Antismoking, 1999).

Nicotine is a precursor of the lung cancer inducer nitrosamine-4(methyl nitrosamino)-1-(3-pyridyl)-1-butanone. The intake of nicotine causes acute symptoms such as miosis, blurred vision, vomiting, dyspnea, increased saliva and secretions from the respiratory tract and decreased heart rate, and chronic symptoms such as arteriosclerosis, hypertension and various circulatory diseases (Brunneman, K. D. et al., J. Natl. Cancer Inst. 89: 868-73 (1996); Carmella, S. G. et al., Cancer Epidemiol Biomarkers Prev. 6 : 113-20 (1997); Wynder, E. L. et al., Environ Health Perspect.103 Suppl 8:143-148(1995)).

Representative of the substances suggested to eliminate or mitigate the toxicity of tar and nicotine is green tea extract (Chung. F. L. Proc. Soc. Exp. Viol. Med. 220: 244-248 (1999); Fujiki, H. et al., Mutat. Res. 18: 307-310 (1998)). A combination of extracts from Houttuynia Cordata, taraxacum officinale Weber, and licorice (Korean Registered Patent No. 0494223) and a combination of extracts from Schizandra chinensis Baillon and Platycodon grandiflorum Nakai(Korean Publicized Patent No. 2004-0102690) are also described as detoxifying tar and nicotine.

Contemplated in accordance with the present invention is the use of an extract from a whole tomato plant, etc. in the detoxifying of the harmful substances in tobacco.

### Disclosure

### Technical Problem

It is an object of the present invention to provide a composition for counteracting the toxicity of smoking.

Other technical features will be apparent from the following description.

### Technical Solution

Leading to the present invention, intensive and thorough research into the detoxification of harmful tobacco substances, conducted by the present inventor, resulted in the finding that each or a mixture of extracts from whole tomato plant, *Hedyotis diffusa,* Sorbus commixta, *Lithospermum erythrorhizon* Siebold & Zucc, and Alnus japonica Steud has the activity of degrading nicotine and neutralizing the toxicity of tar, as identified in the following working examples and experiments where the activity of nicotine degradation was assayed in vitro according to the Barlow et al. method [Barlow R. D, Stone R. B., Clin. Chim. Acta., 165, pp45, 1987] and in animals by monitoring blood nicotine levels, and the activity of tar neutralization was assayed using the Brunnemann et al. method [Brunnemann, K.D. and Hoffiman D., Toxcol, 21, 235(91)]. In addition, a beverage comprising all of the five extracts was prepared and subjected to a sensory test. Improvements in foul breath, phlegm, cough and nausea was obtained from most of the test subjects.

Based on the results of the tests, the present invention addresses a composition for counteracting the toxicity of smoking in accordance with an aspect thereof.

The composition for counteracting the toxicity of smoking according to the present invention includes as an active ingredient at least one selected from among a whole tomato plant extract, an *Hedyotis diffusa* extract, a Sorbus commixta extract, a *Lithospermum erythrorhizon* Siebold & Zucc extract and an Alnus japonica Steud extract.

As used herein, the term "toxicity of smoking" is intended to refer to the toxicity present in nicotine and/or tar. Accordingly, the toxicity of smoking may be used interchangeably with the toxicity of nicotine and/or the toxicity of tar.

As used herein, the term "active ingredient" means a component that can exhibit desired activity, alone or in combination with a carrier, which is itself not active.

The term "whole tomato plant extract," as used herein, is intended to include not only an extract from any part of a tomato plant, such as the leaves, stems, flowers, roots or a combination thereof, by use of a solvent such as methanol, ethanol, acetone, ethylacetate, saturated n-butanol, chloroform, methylene chloride, water or a combination thereof, irrespective of extraction methods, but also a fraction of the extract in such a solvent. So long as it includes the immersion of the extraction target, such as tomato leaves, stems, flowers, roots or combination thereof, in a solvent, any extraction method may be used in the present invention. Examples of the extraction method include cold precipitation, refluxing, warming, and ultrasonication. Preferably, the "whole tomato plant extract" is obtained by immersing the extraction target selected from the group consisting of tomato leaves, stems, flowers, roots and combinations thereof in a solvent selected from the group consisting of water, ethanol or a combination thereof to give an extract of a concentrated liquid phase or a solid phase from which the solvent is removed.

The term *"Hedyotis diffusa* extract" is defined in the same manner as "whole tomato plant extract," with the exception that the extraction target includes Hedyotis diffusa leaves, stems, flowers, roots and combinations thereof.

Likewise, the term *"Lithospermum erythrorhizon* Siebold & Zucc extract" is defined in the same manner as "whole tomato plant extract," with the exception that the extraction target includes *Lithospermum erythrorhizon* Siebold & Zucc leaves, stems, flowers, roots and combinations thereof. The "Sorbus commixta extract" can be defined in the same manner as "whole tomato plant extract," with the exception that the extraction target includes Sorbus commixta leaves, stems, flowers, roots and combination thereof. The "Alnus japonica Steud extract" is also defined in the same manner as "whole tomato plant extract," with the exception that the extraction target includes Alnus japonica Steud leaves, stems, flowers, roots and combinations thereof.

Other terms that are not defined separately herein adhere to the meanings found in dictionaries or generally accepted in the art.

Any amount of the active ingredient such as a whole tomato plant extract and the like may be contained in the composition for counteracting the toxicity of smoking in accordance with the present invention so long as it effectively works to counteract the toxicity of smoking. The effective amount of the active ingredient varies depending on the use, formula, and formulation purpose of the composition, and is generally on the order of 0.001 % by weight to 99.900 % by weight based on the total weight of the composition. By the term "effective amount" is meant the amount of the active ingredient sufficient that results in prevention, amelioration, treatment or delay of the toxicity. The amount is experimentally determined within the ordinary ability of a person skilled in the art.

In addition to the active ingredient such as a whole tomato plant extract, a supplemental ingredient in the composition for counteracting the toxicity of smoking in accordance with the present invention may include natural products or compounds known to detoxify harmful tobacco substances.

Examples of such natural products or compounds include powders or extracts of green tea, black bean, taraxacum officinale Weber, Lonicera japonica, Saururus chinensis (Lour) Baill, cassia seeds, licorice, crab apple, chinese lovage, acanthopanax, Pleuropterus multiflorus TURCZ, Angelica decursiva (Miq.) Frabch & Sav, clove, mandarin peel, Raphanus sativus, Platycodon grandiflorum Nakai, aloe, Schizandra chinensis, Astragalus membranaceus, onion, and stevia. Herein, the extract is as defined for the whole tomato plant extract, with the exception of the extraction target. The extract may be obtained from individual targets or a mixture of the targets.

According to one embodiment, the composition of the present invention may be a food composition for, for example, beverages, gum, jelly, etc.

The food composition may contain an additive such as a sweetener, a flavoring agent, a physiologically active substance, a mineral, etc. in addition to the active ingredient.

A sweetener is used to give a sweet taste to the composition and may be natural or synthetic. Preferable is a natural sweetener. Examples of the natural sweetener include corn syrup, honey, sucrose, fructose, lactose, maltose and other sugars.

A flavoring agent is adopted to enhance the taste or flavor of the composition and may be natural or synthetic. Preferable is a natural flavoring agent. A flavoring agent, if natural, may have the function of nutritional supplementation in addition to enhancing the flavor. Examples of the natural flavoring agent include those obtained from apple, lemon, mandarin, grape, strawberry, peach, green tea leaves, Polygonatum odoratum, bamboo leaves, cinnamon, chrysanthemum leaves, and/or jasmine. Other natural flavoring agents include those from ginseng (red ginseng), bamboo shoots, aloe vera, and/or ginkgo nuts. The natural flavoring agent may be in the form of a liquid concentrate or a solid extract. A synthetic flavoring agent may be used, and is exemplified by esters, alcohols, aldehydes and terpenes.

Among the physiological active substance are catechins, such as catechin, epicatechin, gallocatechin and epigallocatechin, and vitamins, such as retinols, ascorbic acid, tocopherol, calciferol, thiamine and rivoflavin.

As for the mineral, examples thereof include calcium, magnesium, chrome, cobalt, copper, fluorides, germanium, iodine, iron, lithium, magnesium, manganese, molybdenum, phosphorus, calcium, selenium, silicone, sodium, sulfur, vanadium, and zinc.

Optionally, the food composition of the present invention may further include a preservative, an emulsifier, an acidulant, and a thickener in addition to the additives such as a sweetener, etc.

These agents such as preservatives, emulsifiers, etc. are used in as minimal an amount as possible to achieve the purpose of their addition. Numerically, their amount ranges from approximately 0.0005 % by weight to 0.5 % by weight based on the total weight of the composition.

Examples of the preservative useful in the present invention include calcium sorbate, sodium sorbate, potassium sorbate, calcium benzoate, sodium benzoate, potassium benzoate, and EDTA (ethylenediaminetetracetic acid).

Acacia gum, carboxymethylcellulose, xanthan gum, and pectin are emulsifiers that can be used in the present invention.

Representative among the acidulants are citric acid, malic acid, fumaric acid, adipic acid, phosphoric acid, gluconic acid, tartaric acid, ascorbic acid and acetic acid. The acidulant may be added for the purpose of suppressing microbial proliferation as well as enhancing the taste.

The thickener useful in the present invention may be exemplified by a suspending agent, a flocculant, a gel forming agent, and a swelling agent.

In accordance with another embodiment, the composition of the present invention may be used as a pharmaceutical composition.

The pharmaceutical composition according to the present invention comprises a pharmaceutically acceptable carrier in addition to the active ingredient and may be formulated into oral dosage forms (tablets, suspensions, granules, emulsions, capsules, syrup, etc.), parenteral dosage forms (sterile injections, aqueous or oily suspensions, etc.), and topical application forms (solutions, creams, ointments, gels, lotions, patches, etc.).

As used herein, the term "pharmaceutically acceptable" means that a material does not interfere with the effectiveness of the biological activity of the active ingredients and is low enough in toxicity to be used on the subject.

Examples of the pharmaceutically acceptable carrier include lactose, glucose, sucrose, starch (e.g., corn starch, potato starch, etc.), cellulose and its derivatives (e.g., sodium carboxymethyl cellulose, ethyl cellulose, etc.), malt, gelatin, talc, a solid lubricant (e.g., stearic acid, magnesium stearate, etc.), calcium sulfate, vegetable oil (e.g., peanut oil, cotton seed oil, sesame oil, olive oil), polyol (e.g., propylene glycol, glycerine), alginic acid, emulsifiers (e.g., TWEENS), wetting agents (sodium lauryl sulfate), colorants, flavoring agents, stabilizers, antioxidants, preservatives, water, saline, and phosphate buffered saline. These carriers may be used, individually or in combination, according to the formulation of the pharmaceutical composition.

A suitable excipient may be also employed in the pharmaceutical composition of the present invention. For example, an excipient suitable for formulating the pharmaceutical composition of the present invention into an aqueous suspension may be a suspending agent or dispersant such as sodium carboxymethyl cellulose, methyl cellulose, hydropropylmethylcellulose, sodium alginate, or polyvinylpyrrolidone. When the pharmaceutical composition is formulated into an injection, Ringer's solution, or isotonic sodium chloride may be used as an excipient.

To administer the pharmaceutical composition of the present invention, an oral route or a parenteral route such as a topical route may be taken.

The daily dose pharmaceutically composition of the present invention may be administered at a daily dose of from 0.001 ∼ 150 mg/kg of body weight and in a single dose or in multiple doses per day. The dose of the pharmaceutical composition of the present invention may vary depending on various factors including the route of administration, the patient's age, gender and weight, and the severity of illness and thus must be in no way understood to limit the scope of the present invention.

### Advantageous Effects

As described hitherto, the composition for counteracting the toxicity of smoking, comprising an extract from whole tomato plant, or other herbs is provided. The composition may be used as a food composition for gum or beverages or as a pharmaceutical composition.

### Description of Drawings

FIG. 1 is a graph showing that the whole tomato plant extract degrades nicotine in a time-dependent manner in vitro.
FIG. 2 is a graph showing that the Hedyotis diffusa extract degrades nicotine in a time-dependent manner in vitro.
FIG. 3 is a graph showing that the Sorbus commixta extract degrades nicotine in a time-dependent manner in vitro.
FIG. 4 is a graph showing that the Lithospermum erythrorhizon Siebold & Zucc extract degrades nicotine in a time-dependent manner in vitro.
FIG. 5 is a graph showing that the Alnus japonica Steud extract degrades nicotine in a time-dependent manner in vitro.

### Mode for Invention

A better understanding of the present invention may be obtained through the following examples which are set forth to illustrate, but are not to be construed as limiting the present invention.

### WORKING EXAMPLES : Preparation of Extract from Whole Tomato Plant, Hedyotis diffusa, Sorbus commixta, Lithospermum erythrorhizon Siebold & Zucc or Alnus japonica Steud.

### WORKING EXAMPLE 1: Preparation of Extract from Whole Tomato Plant

1 kg of sliced whole tomato plant (leaves, stems and roots) was immersed in 5 ℓ of 50 % ethanol at 50°C for 10 hours, followed by removal of the plant debris. The solution was concentrated at a reduced pressure to remove the solvent and to afford an extract in a solid phase.

### WORKING EXAMPLE 2 : Preparation of Extract from Hedyotis diffusa

1 kg of sliced Hedyotis diffusa (leaves, stems and roots) was immersed in 5 ℓ of 50 % ethanol at 50°C for 10 hours, followed by removal of the plant debris. The solution was concentrated at a reduced pressure to remove the solvent and to afford an extract in a solid phase.

### WORKING EXAMPLE 3 : Preparation of Extract from Sorbus commixta

1 kg of sliced Sorbus commixta (leaves, stems and roots) was immersed in 5 ℓ of 50 % ethanol at 50°C for 10 hours followed by removal of the plant debris. The solution was concentrated at a reduced pressure to remove the solvent and to afford an extract in a solid phase.

### WORKING EXAMPLE 4 : Preparation of Extract from Lithospermum erythrorhizon Siedold & Zucc

1 kg of sliced Lithospermum erythrorhizon Siedold & Zucc (leaves, stems and roots) was immersed in 5 ℓ of 50 % ethanol at 50°C for 10 hours followed by removal of the plant debris. The solution was concentrated at a reduced pressure to remove the solvent and to afford an extract in a solid phase.

### WORKING EXAMPLE 5 : Preparation of Extract from Alnus japonica Steud

1 kg of sliced Alnus japonica Steud (leaves, stems and roots) was immersed in 5 ℓ of 50 % ethanol at 50°C for 10 hours followed by removal of the plant debris. The solution was concentrated at a reduced pressure to remove the solvent and to afford an extract in a solid phase.

### PREPARATION EXAMPLE: Preparation of Mixed Beverage

A drink was prepared by mixing the extracts of WORKING EXAMPLES 1 to 5 in amounts listed in Table 1, below.

**TABLE 1**

| Components and Contents of Beverage | |
|---|---|
| | Content (wt%) |
| Extract of WORKING EXAMPLE 1 | 2.0 |
| Extract of WORKING EXAMPLE 2 | 2.0 |
| Extract of WORKING EXAMPLE 3 | 2.0 |
| Extract of WORKING EXAMPLE 4 | 2.0 |
| Extract of WORKING EXAMPLE 5 | 2.0 |
| Honey | 10.0 |
| Grapefruit seed extract (natural preservative) | 1.0 |
| Distilled water | q.s. (to 100) |

### EXPERIMENTAL EXAMPLES: Assay for Capability of Degrading Nicotine and Neutralizing the Toxicity of Tar and Sensory Test

### EXPERIMENTAL EXAMPLE 1: Assay for Nicotine Degradation

### Experimental Example 1-1 In vitro Assay

Each of the extracts of the WORKING EXAMPLES was assayed for ability to degrade nicotine according to the Barlow et al. method [Barlow R. D, Stone R. B., Clin. Chim. Acta., 165, pp45, 1987] .

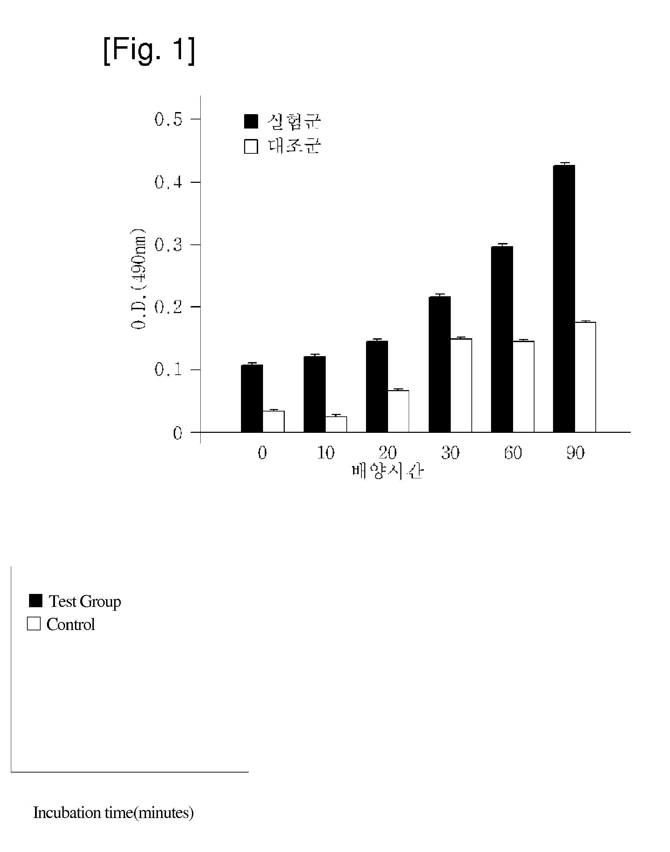

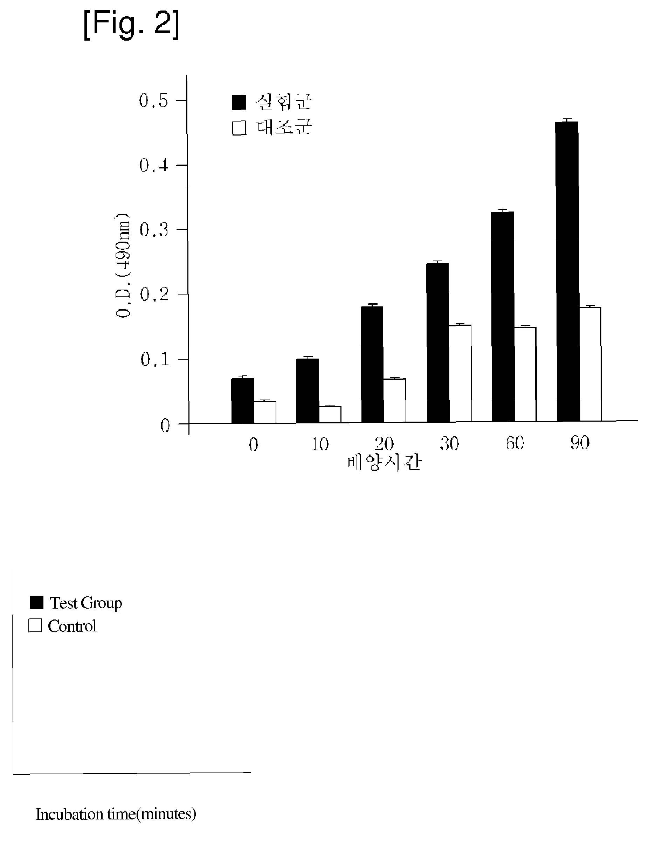

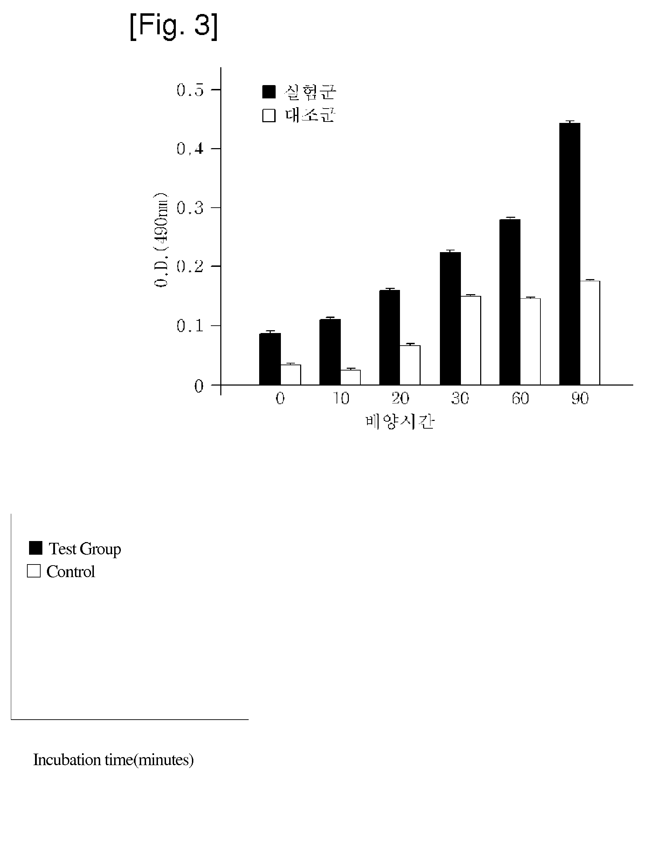

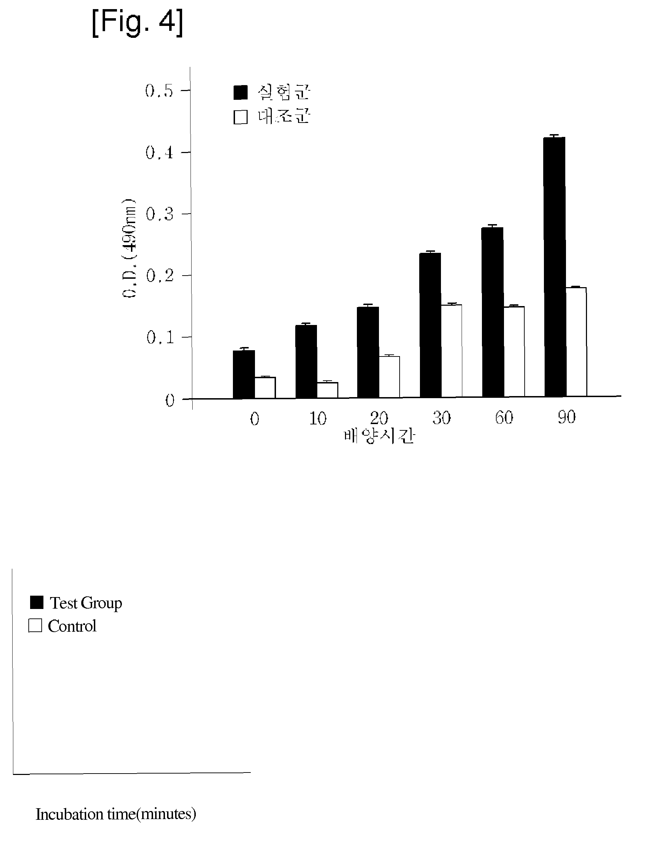

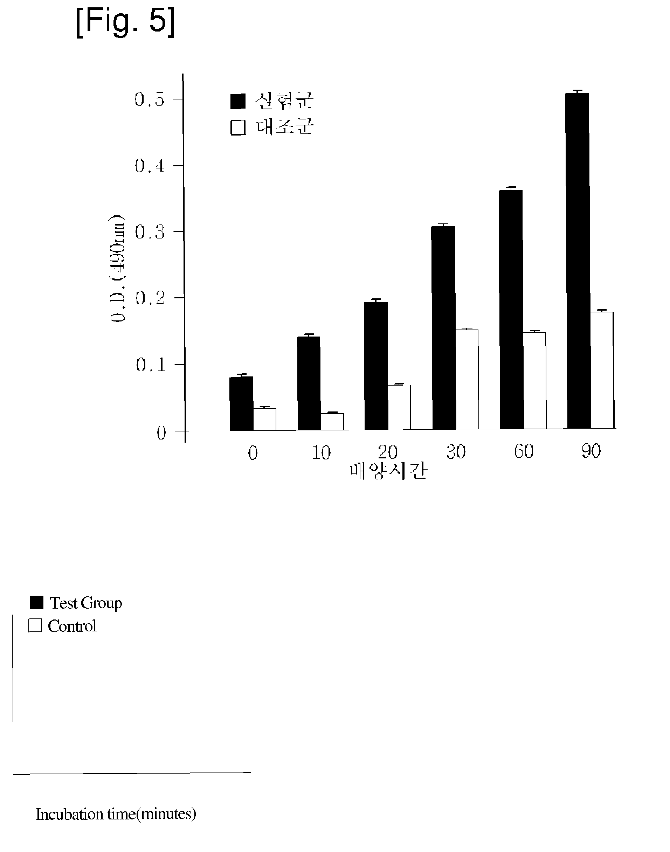

Together with 200 *µ*ℓ of 1 mM nicotine (Sigma Co., USA), the same volume of one of the extracts (10 % (w/v) in distilled water) was placed in a microtube (5 mℓ). During incubation at 25°C, cotinine, which is a metabolite of nicotine, was quantified at predetermined times (0, 10, 20, 30, 60 and 90 minutes). As a control, a mixture of *200 µ*ℓ of 1 mM nicotine, and 200 *µ*ℓ of distilled water were used instead of the extract. Then, 100 µℓ of 4M sodium acetate buffer (pH4.7), *40 µ*ℓ of 1.5M potassium cyanate, *40 µ*ℓ of 0.4 M chloramine-T, and *200 µ*ℓ of 78mM barbituric acid in 50 % (v/v) acetonitrile were added in that order to a microtube, mixed for 10 seconds, and incubated for 15 minutes at room temperature, followed by terminating the reaction with 40 *µ*ℓ of 1M sodium metabisulphite. Absorbance at 490 nm determined the quantity of cotinine which changed with time. All experiments were performed independently in triplicate and the results are expressed as Mean±SD in FIGS. 1 to 5. With reference to FIGS. 1 to 5, all of the extracts of the WORKING EXAMPLES converted nicotine to cotinine in a time dependent manner.

### Experimental Example 1-2: Animal Assay

Six-week-old, specific pathogen-free, Spraque-Dawley rats, each weighing 260∼280 g, were purchased from Central Lab. Animal Inc. Korea. They were divided into six groups of five in respective cages and acclimated for 2 weeks to a breeding room maintained at 21±2°C under a light/dark cycle of 12L/12D, and were fed sufficient amounts of water and solid food.

Of the six groups, one was used as a control while the remaining five groups were used as test groups. Each extract was orally administered as a mixture with the solid food to the rats of test groups at a dose a dose of 1.0 g/kg/day for 7 days while nicotine was intraperitoneally administered at a dose of 3 mg/kg. The control group were fed but the food did not contain the extract and were injected intraperitoneally with nicotine at a dose of 3 mg/kg. Thereafter, 1 mℓ of blood was taken from the heart and used to measure the blood nicotine level. In this regard, the blood was adjusted to pH 9.0 with diluted ammonia water, maintained for 15 minutes in an Extrelut column(Merck Co.) and eluted with 15 mℓ of ethyl acetate. The eluted acetate was removed by reducing pressure and distillation to form a powder which was then dissolved in *100 µ*ℓ of ethyl acetate and used as an analysis sample. Analysis was performed using a gas chromatography-mass spectrometer (FISONS Instruments Co.).

Blood nicotine levels are expressed as Mean±SD in Table 2, below.

**TABLE 2**

| | Blood Nicotine Level |
|---|---|
| Group | Blood Nicotine Level (*µ*g/mℓ) |
| Test Group 1 | 1.20±0.13 |
| Test Group 2 | 1.74±0.15 |
| Test Group 3 | 1.72±0.09 |
| Test Group 4 | 1.93±0.12 |
| Test Group 5 | 1.64±0.13 |
| Control | 2.28±0.12 |
| * Test groups 1 to 5 were administered with the extracts of Examples 1 to 5, respectively. | |

### EXPERIMENTAL EXAMPLE 2: Assay for Capability of Neutralizing the Toxicity of Tar

The extracts of the WORKING EXAMPLES were assayed for capability of neutralizing the toxicity of tar according to the Brunnemann et al. method [Brunnemann, K.D. and Hoffiman D., Toxcol, 21, 235(91)].

Test animals were divided into groups of five. They were injected intraperitoneally with tar at a dose of 30 mg/kg/10mℓ while being orally administered with the extracts of the Examples, respectively, at a daily dose of 1.0 g/kg/day for 7 days. Controls were administered with distilled water instead of the extracts. Their survival was monitored.

Dead animals were counted for 7 days and the results are summarized in Table 3, below.

**TABLE 3**

| Count of Dead Rats | | | | | | | | |
|---|---|---|---|---|---|---|---|---|
| Group | Dead Count | | | | | | | Total Count of Survived |
| | Day 1 | Day 2 | Day 3 | Day 4 | Day 5 | Day 6 | Day 7 | |
| Test Group 1 | 0 | 0 | 0 | 0 | 0 | 0 | 0 | 5 |
| Test Group 2 | 0 | 0 | 0 | 0 | 0 | 1 | 0 | 4 |
| Test Group 3 | 0 | 0 | 0 | 0 | 0 | 1 | 1 | 3 |
| Test Group 4 | 0 | 0 | 0 | 0 | 1 | 1 | 0 | 3 |
| Test Group 5 | 0 | 0 | 0 | 0 | 0 | 1 | 1 | 3 |
| Control | 0 | 0 | 1 | 0 | 1 | 1 | 1 | 1 |

### EXPERIMETAL EXAMPLE 3: Sensory Test

The effect of the beverage of the Preparation Example on smoking symptoms was examined.

Each of 100 smokers, which had smoked one or more packs a day, was orally administered with the beverage at a dose of 140 mℓ twice a day for one month.

In the sensory test, the effect of the beverage on foul breath, phlegm, cough and nausea was evaluated using a five-point scale method (5: greatly improved, 4: significantly improved, 3: moderately improved, 2: slightly improved, and 0: not improved). The results are summarized in Table 4, below.

**TABLE 4**

| Counteracting Effect on Nicotine (unit: person) | | | | | |
|---|---|---|---|---|---|
| | Greatly Improved | Significantly Improved | Moderately Improved | Slightly Improved | Not Improved |
| Foul breath | 72 | 18 | 4 | 4 | 2 |
| Phlegm | 68 | 21 | 6 | 3 | 2 |
| Cough | 54 | 26 | 10 | 5 | 5 |
| Nausea | 66 | 14 | 14 | 4 | 2 |

As can be seen in the data, the beverage of the present invention can greatly improve the smoking symptoms.

### Sequence List Free Text

None

## Claims

1. A composition for counteracting toxicity of smoking, including as an active ingredient at least one selected from a whole tomato plant extract, an Hedyotis diffusa extract, a Sorbus commixta extract, a Lithospermum erythrorhizon Siebold & Zucc extract and an Alnus japonica Steud extract.

2. The composition of claim 1, wherein the whole tomato plant extract is prepared by subjecting tomato leaves, stems, flowers, roots or a combination thereof to extraction with a solvent selected from among methanol, ethanol, acetone, ethyl acetate, saturated normal-butanol, chloroform, methylene chloride, water and a combination thereof.

3. The composition of claim 1, wherein the whole tomato plant extract is prepared by subjecting tomato leaves, stems, flowers, roots or a combination thereof to extraction with a solvent selected from among ethanol, water and a combination thereof.

4. The composition of claim 1, wherein the Hedyotis diffusa extract is prepared by subjecting Hedyotis diffusa leaves, stems, flowers, roots or a combination thereof to extraction with a solvent selected from among methanol, ethanol, acetone, ethyl acetate, saturated normal-butanol, chloroform, methylene chloride, water and a combination thereof.

5. The composition of claim 1, wherein the Hedyotis diffusa extract is prepared by subjecting Hedyotis diffusa leaves, stems, flowers, roots or a combination thereof to extraction with a solvent selected from among ethanol, water and a combination thereof.

6. The composition of claim 1, wherein the Sorbus commixta extract is prepared by subjecting Sorbus commixta leaves, stems, flowers, roots or a combination thereof to extraction with a solvent selected from among methanol, ethanol, acetone, ethyl acetate, saturated normal-butanol, chloroform, methylene chloride, water and a combination thereof.

7. The composition of claim 1, wherein the Sorbus commixta extract is prepared by subjecting Sorbus commixta leaves, stems, flowers, roots or a combination thereof to extraction with a solvent selected from among ethanol, water and a combination thereof.

8. The composition of claim 1, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among methanol, ethanol, acetone, ethyl acetate, saturated normal-butanol, chloroform, methylene chloride, water and a combination thereof.

9. The composition of claim 1, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof.

10. The composition of claim 1, wherein the Alnus japonica Steud extract is prepared by subjecting Alnus japonica Steud leaves, stems, flowers, roots or a combination thereof to extraction with a solvent selected from among methanol, ethanol, acetone, ethyl acetate, saturated normal-butanol, chloroform, methylene chloride, water and a combination thereof.

11. The composition of claim 1, wherein the Alnus japonica Steud extract is prepared by subjecting Alnus japonica Steud leaves, stems, flowers, roots or a combination thereof to extraction with a solvent selected from among ethanol, water and a combination thereof.

12. The composition of claim 1, including a combination of a whole tomato plant extract, an Hedyotis diffusa extract, a Sorbus commixta extract, a Lithospermum erythrorhizon Siebold &Zucc extract and an Alnus japonica Steud extract as an active ingredient.

13. The composition of claim 1, further including at least one selected from among powders or extracts of green tea, black bean, taraxacum officinale Weber, Lonicera japonica, Saururus chinensis(Lour) Baill, cassia seeds, licorice, crab apple, chinese lovage, acanthopanax, Pleuropterus multiflorus TURCZ, Angelica decursiva (Miq.) Franch & SAv, clove, mandarin peel, Raphanus sativus, Platycodon grandiflorum Nakai, aloe, Schizandra chinensis Baillon, Astragalus membranaceus BUNGE, onion, and stevia.

14. The composition of any one of claims 1 to 13, wherein the composition is a food composition.

15. The composition of claim 14, wherein the food composition is in a form of beverage, gum or jelly.

16. The composition of any one of claims 1 to 13, wherein the composition is a pharmaceutical composition.
